# EUROPEAN PATENT APPLICATION

(11) **EP 2 597 044 A1**
(43) Date of publication of application: **29.05.2013**
(21) Application number: 11009355.6
(22) Date of filing: 25.11.2011
(51) Int. Cl.: B65D 1/08, B65D 47/18, A61F 9/00, B01L 3/02

(54) **Drop-dispensing insert and container with a drop-dispensing insert**

(71) Applicant: Hoffmann Neopac AG, 3602 Thun (CH)
(72) Inventor: Geiger, Andreas, 3612 Steffisburg (CH); Kubesch, Christian, 3672 Oberdiessbach (CH); Mathys, Jan, 3672 Oberdiessbach (CH)
(74) Representative: Fischer, Britta Ruth

(57) **Abstract**

The present invention relates to a drop-dispensing insert (2) for a container (1), comprising a delivery channel (5) with an opening (6) at its distal end (7), the opening (6) determining the size of a dispensed drop, a neck (9) for attaching the insert (2) to a container body, and throttling means (11, 12), wherein the throttling means comprise a projecting element (11) that is situated at the proximal end (8) of the delivery channel (5) such that its longitudinal axis is substantially aligned with the longitudinal axis of the delivery channel (5), the projecting element (11) projecting into a hollow space (10) formed by the neck (9) and having a groove (12) that connects the hollow space (10) with the delivery channel (5), wherein the outer diameter of at least a part of the projecting element (11) matches substantially the inner diameter of an outlet channel (13) of the container body to which the insert (2) is to be attached.

## Description

The invention relates to a drop-dispensing insert and to a container with such a drop-dispensing insert for controllably dispensing liquid contents as drops. The container is in particular made of flexible plastic material such that when a user gently squeezes the container body one or more drops are dispended. The container may be for example a tube or a bottle. Typical application areas are among others in pharmaceutics and cosmetics.

It is often required to dispend a liquid in the form of drops. This is for example often required for the administration of medication such as eye drops to a user's or patient's eye. As the amount of medication is typically defined by the number of drops it is essential that the volume of the drops is substantially kept constant independently of the type of liquid, its state and the pressure that is applied to the container body by the user.

From patent document DE-C1-101 12 332 there is known a drop cap for a squeezable container. Before the inlet opening of the delivery channel there is provided a throttling device in the flow direction in the cap body to ensure that a predetermined drop volume is maintained in particular with large drops. The throttling device is given by a permeable opening in a separating wall that is arranged perpendicularly to the flow direction.

European patent application EP-A1-0 362 911 discloses a bottle for controllably dispensing liquid contents as drops that has a delivery tip portion with an outwardly opening passage. The delivery tip portion has at its narrow end an insert element with an internal bore of selected diameter and length for thereby regulating the rate of flow of liquid contents to the tip portion.

From the international patent application WO-A1-2004/069679 a container with a drop dispenser with a outwardly opening delivery passage is known. The drop dispenser comprises a spout element and an insert element that is arranged at the narrow end of the delivery passage. The insert element has a lateral notch, a transversal notch and a central bore. A throttling passage is formed between the insert element and the spout element in that the insert element is surrounded by the inside walls of the spout element. The notches of the insert element form together with the inside walls of the spout element the channels of the throttling passage. When the liquid in the container is pressurized it passes through the notches to the bore and into the delivery passage.

International patent application WO-A1-2004/069678 shows a container with a drop-dispensing insert that comprises an outwardly opening delivery passage and two throttling passages that enter laterally and diametrically opposed into the lower end portion of the delivery passage. The insert has a shaft which can be attached to a cylindrical opening or socket of the container body for fixing it to the container body. At the container side end of the throttling passages the diameter of the insert is smaller than the diameter of the opening of the container body, such that liquid can pass between the inner wall of the opening and the outside of the drop-dispending insert to the throttling passage.

It is an object of the invention to provide a drop-dispensing insert for a container and a container for controllably dispensing liquid contents therefrom as regular drops without any jet. It is a further object of the invention to provide a drop-dispensing insert for a container and a container for dispensing liquid contents in the form of drops whose size is basically independent of the pressure exerted on the container.

In order to implement these and still further objects of the invention, which will become more readily apparent as the description proceeds, a drop-dispensing insert (in the following also called in short "insert") for a container is provided that comprises a delivery channel with an opening at its distal end, the opening determining the size of the dispensed drops, a neck for attaching the insert to a container body and throttling means. The throttling means comprise a projecting element that is situated at the proximal end of the delivery channel such that its longitudinal axis is substantially aligned with the longitudinal axis of the delivery channel. The projecting element projects into the hollow space that is formed by the neck. Furthermore, the projecting element has a groove that connects this hollow space with the delivery channel. The outer diameter of at least a part of the projecting channel matches substantially the inner diameter of an outlet channel of the container body to which the insert is to be attached.

The distal end of the delivery channel is that end from which the drops are dispensed. The proximal end of the delivery channel is that end opposite to the distal end, i.e. the end closest to the container body when assembled. The outer diameter of the projecting element and the inner diameter of the outlet channel of the container body are measured in the direction perpendicular to the longitudinal axis of the projecting element and to the longitudinal axis of the container body, respectively. The longitudinal axis of the respective components (i.e. projecting element, delivery channel, container body) is that axis that is in the direction of flow when the container assembled and used.

The groove of the projecting element is preferably a longitudinal groove that extends in particular in the same direction as the longitudinal axis of the projecting element. The groove delays/hinders/throttles the flow of the liquid during dispensing, thereby aiding in the forming of the drops. The groove is preferentially arranged in the surface of the projecting element and runs preferably along its entire length in the longitudinal direction. The groove can be given by a bore. The groove has preferably a diameter in the range of substantially 0.05 mm to 0.1 mm, in particular a diameter of substantially 0.1 mm. The groove can be formed by injection moulding together with the entire insert. The groove can also be formed by different measures as e.g. laser drilling. The insert, in particular its dimensions and the dimensions of the groove, can be chosen with respect to different fluid viscosities and required drop sizes.

The container according to the invention comprises a drop-dispensing insert according to the invention and a container body with a head that has an outlet channel. The insert is attached to the head of the container body. The projecting element of the insert extends, preferably only partly, into the outlet channel and the inner diameter of the outlet channel substantially matches the outer diameter of that part of the projecting element that extends into the outlet channel. The hollow space formed by the neck of the insert is preferably only partly filled by the head of the container such that some hollow space remains. The container body can as well as the insert be manufactured by injection moulding.

With the insert and the container of the invention it can advantageously be achieved that the dispensed drops have substantially the same size even if the pressure exerted on the container body varies.

Further advantageous features and applications of the invention can be found in the dependent claims as well as in the following description of the drawings illustrating the invention. In the drawings like reference signs designate the same or similar parts throughout the several figures of which:
Fig. 1 shows an assembled cross-sectional view in elevation of the upper part of a container according to the invention,
Fig. 2 shows a cross-sectional view in elevation of an insert according to the invention,
Fig. 3 shows a cross-sectional view in elevation of the head of the container body of a container according to the invention, and
Fig. 4 shows a circular section of a plan view of a container according to the invention.

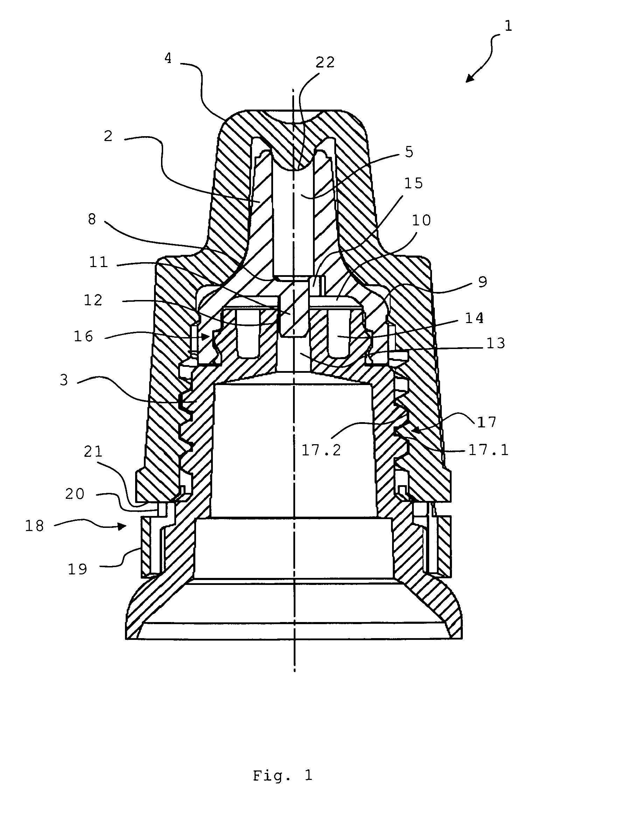

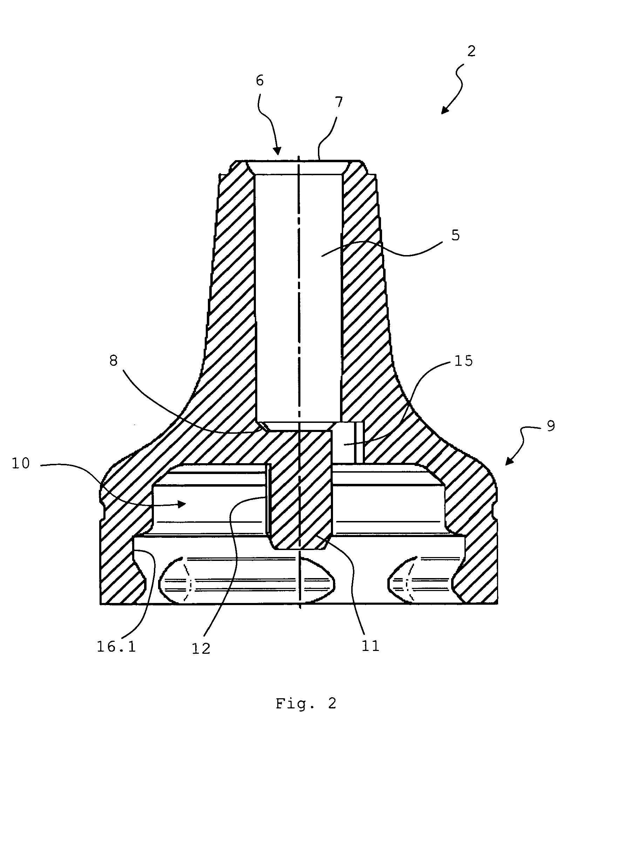

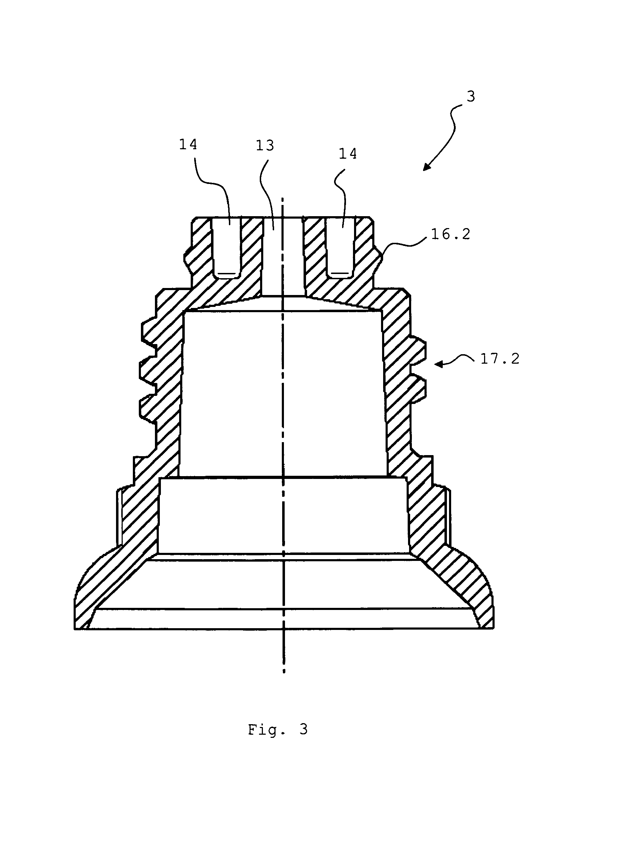

Figure 1 shows a container 1 according to the invention with a removable drop-dispensing insert 2 (in short: insert) according to the invention and the head 3 of the body of the container (also called container body), the container body containing the liquid to be dispensed. The remaining part of the container body is not shown for simplicity. The insert 2 is in detail depicted in Figure 2. The head 3 of the container body is in detail depicted in Figure 3. The container 1 further comprises a removable cap 4 that can be placed over the insert 2 and connected to the head 3 of the container body for closing the container 1. Figure 1 depicts the closed state of the container 1. The container 1 may have the form of a bottle or a tube made of flexible material. The insert 2, the container body and the cap 4 are preferably made from flexible plastic material using injection moulding.

The drop-dispensing insert 2 comprises a delivery channel 5 that has an opening 6 at its distal end 7. The size of the opening 6 basically determines among others the size of the dispensed drops. The delivery channel 5 is preferably cylindrical but may also take other forms if appropriate such as a conical form where the opening 6 at the distal end 7 is wider than the opening at the proximal end 8.

The drop-dispensing insert 2 further comprises a neck 9 for attaching the insert 2 to the head 3 of a container body. The neck 9 basically begins at the proximal end 8 of the delivery channel 5 and extends away (downward in Figures 2 and 3) from it. It is open toward a container body it shall be connected to, i.e. in the direction opposite to the distal end 7 of the delivery channel 5. The neck 9 forms a hollow space 10 with its inner surface.

The drop-dispensing insert 2 furthermore has throttling means that comprise a projecting element 11. The projecting element 11 is situated at or below, respectively, the proximal end 8 of the delivery channel 5 with the longitudinal axis of the projecting element 11 and the longitudinal axis of the delivery channel 5 being preferably aligned. The projecting element 11 extends into the hollow space 10 formed by the neck 9. The projecting element 11 is preferably cylindrical in shape with its outer diameter being preferentially equal to or slightly smaller than the inner diameter of the delivery channel 5.

The projecting element 11 has a groove 12 that connects the hollow space 10 with the delivery channel 5, in particular with its proximal end 8. The groove 12 is preferably given by a bore. The groove 12 is preferentially longitudinal and preferentially arranged on the outer surface of the projecting element 11. It preferably runs along the entire length of the projecting element 11 in the longitudinal direction. The groove 12 may also pass partly or entirely through the projecting element 11. It may also be bended. It preferably has a diameter in the range of 0.05 mm to 0.1 mm depending on the viscosity of the liquid to be dispensed. The shape of the groove 12 and its diameter, respectively, can be chosen in dependence on the viscosity of the liquid contents of the container body and/or on the desired drop size/volume.

The head 3 of the container body has an outlet channel 13 for the liquid. At least the lower part of the projecting element 11 extends into this outlet channel 13 when the container 1 is assembled. Preferably not the entire projecting element 11 is pushed into the outlet channel 13 but only its lower part such that there remains some hollow space 10 when the insert 2 is attached to the container body. With the lower part of the projecting element 11 that part is meant that is further away from the proximal end 8 of the delivery channel 5. The inner diameter of the outlet channel 13 substantially corresponds to/matches the outer diameter of preferably the entire part of the projecting element 11 that is inside the outlet channel 13 when assembled. By this it is achieved that the liquid contents of the container body has to pass through the groove 12 of the projecting element, i.e. through a passage defined by the groove 12 and the inner wall of the outlet channel 13, thereby slowing down/throttling the flow of the liquid such that dispensing the liquid contents in form of a jet or spurt can be avoided and the liquid contents can advantageously be dispensed in form of regular drops whose size/volume does not depend on the pressure exerted on the container body by a user who wants to dispense some of its liquid contents.

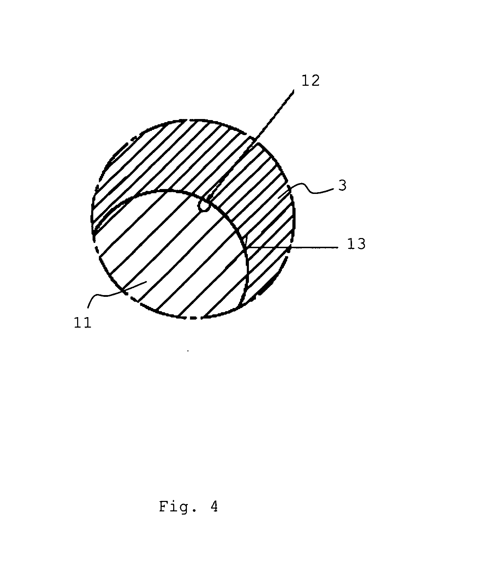

Figure 4 shows part of a cross-sectional view in plan of the projecting element 11 extending into the outlet channel 13 of the head 3. It can be seen that the groove 12 forms with the inner wall of the outlet channel 13 the only passage through which the liquid contents of the body container can pass when it is squeezed out of the pressurized body container. The outlet channel 13 may e.g. have a diameter of substantially 1.5 mm, with the groove 12 having a diameter in the range of 0.05 mm to 0.1 mm.

The head 3 of the container body may comprise an annular recess 14 to safe material, the annular recess 14 encircling the outlet channel and being open toward the insert when assembled.

The drop-dispensing insert 2 according to the invention preferably comprises one or more collecting chambers 15 that are situated around the upper part of the projecting element 11. The upper part of the projecting element 11 is that part closer to the proximal end 8 of the delivery channel 5. The collecting chambers 15 are open toward the hollow space 10 and the delivery channel 5. If several collecting chambers 15 are provided they are preferably equally distributed and equally spaced around the projecting element 11 with each collecting chamber 15 having preferentially the form of a section of a ring and each collecting chamber 15 being preferentially of equal size. In particular, three collecting chambers 15 are provided. With the groove 12 being provided on the surface of the projecting element 11, there is preferably no collecting chamber 15 adjoining to that part of the surface of the projecting element 11 that comprises the groove 11.

For dispensing its liquid contents the container 1 is held with the opening 6 of the insert 2 downward, i.e. toward the geometrical centre of the earth. After dispensing, when turning the container 1 upside down (i.e. into the position depicted in Figure 1), liquid that may be inside the delivery channel 5 and now glides down its surface can be collected by the collecting chambers 15 and via the collecting chambers 15 by the hollow space 10. Furthermore, during dispensing liquid can enter the hollow space 10 from the groove 12. This liquid can then be gathered by the collecting chambers 15.

The container 1 comprises first connecting means 16 for connecting the insert 2 with the head 3 of the container body. The first connecting means 16, 16.1, 16.2 are provided on the outside of the head 3 of the container body and on the inside of the neck 9 of the insert 2. In particular, the first connecting means 16 are formed as snap-on connection means comprising an annular recess 16.1 on the inside of the neck 9 of the insert 2 and an annular bulge 16.2 on the outside of the head 3 of the container body. The bulge 16.2 engages with the recess 16.1 for connection of the insert 2 with the head 3. Of course, the arrangement of the snap-on connection means can also be vice versa in that the annular bulge is situated at the inside of the neck 9 and the annular recess is situated at the outside of the head 3. The first connecting means 16 may also be realized differently e.g. as screw connection means with appropriate threads on the inside of the neck 9 and the outside of the head 3.

Second connecting means 17 are provided for connecting the cap 4 to the head 3 of the container body. The second connecting means 17 are preferably formed as screw connection means but may also be realized differently e.g. as snap-on connection means with an annular recess and an annular bulge as described with respect to the first connection means 16. If the second connecting means 17 are realized as screw connection means then there is provided a external (male) thread 17.2 on the outside of the head 3 before the first connecting means 16.2 in the direction of flow when dispensing and an internal (female) thread 17.1 on the inside of that part of the cap 4 that overlaps the external tread 17.2 in a closed state of the container 1. The cap 4 preferably has on its inside a dent 22 that fits into and seals the opening 6 of the insert 2 in a closed state of the container 1.

Moreover, the container 1 according to the invention comprises means 18 for guaranteeing a first opening, i.e. the container 1 is tamper-proof. The means 18 for guaranteeing a first opening comprise a ring 19 that is arranged substantially unrotatably around the head 3 of the container body, in particular around a part of the head 3 that is located before the second connecting means 17.2 in the direction of flow during dispensing. The ring 19 is connected via bars 20 to the rim 21 of the cap 4 such that the connection between the rim 21 and the ring 19 breaks when the cap 4 is rotated by a user, thereby providing a first opening guarantee.

## Claims

1. A drop-dispensing insert for a container (1), in particular a tube, the insert (2) comprising
- a delivery channel (5) with an opening (6) at its distal end (7), the opening (6) determining the size of a dispensed drop,
- a neck (9) for attaching the insert (2) to a container body,
- throttling means (11, 12),
**characterized in that** the throttling means comprise a projecting element (11) that is situated at the proximal end (8) of the delivery channel (5) such that its longitudinal axis is substantially aligned with the longitudinal axis of the delivery channel (5), the projecting element (11) projecting into a hollow space (10) formed by the neck (9) and having a groove (12) that connects the hollow space (10) with the delivery channel (5), wherein the outer diameter of at least a part of the projecting element (11) matches substantially the inner diameter of an outlet channel (13) of the container body to which the insert (2) is to be attached.

2. The insert according to claim 1, wherein the groove (12) is a longitudinal groove, in particular a longitudinal bore, in the surface of the projecting element (11) that runs along the entire length of the projecting element (11).

3. The insert according to claim 1 or 2,
wherein the groove (12) has diameter in the range of substantially 0.05 to 0.1 mm.

4. The insert according to one of the preceding claims, wherein one or more collecting chambers (15) are provided around the projecting element (11), the collecting chambers (15) being open toward the hollow space (11) and the delivery channel (5).

5. The insert according to claim 4, wherein the one or more collecting chambers (15) are arranged equally spaced around the projecting element (11), with in particular three collecting chambers (15) being provided.

6. A container with a container body whose head (3) has an outlet channel (13), **characterized by** a drop-dispensing insert (2) according to one of the preceding claims that is attached to the head (3) of the container body, wherein the projecting element (11) of the insert (2) extends at least partly into the outlet channel (13) and the inner diameter of the outlet channel (13) substantially matches the outer diameter of that part of the projecting element (11) that extends into the outlet channel (13).

7. The container according to claim 6,
wherein the hollow space (10) formed by the neck (9) of the insert (2) is only partly filled by the head (3) of the container body.

8. The container according to claim 6 or 7,
wherein first connecting means (16, 16.1, 16.2) are provided on the outside of the head (3) of the container body and on the inside of the neck (9) of the insert (2) for connecting the insert (2) with the head (3) of the container body.

9. The container according to claim 8,
wherein the first connecting means (16, 16.1, 16.2) are formed as snap-on connection means.

10. The container according to one of the claims 6 to 9, wherein the inner diameter of the outlet channel (15) is substantially 1.5 mm.

11. The container according to one of the claims 6 to 10, wherein a cap (4) is provided that is placed over the insert (2) and connected by second connecting means (17, 17.1, 17.2) with the head (3) of the container body in a closed state of the container (1).

12. The container according to claim 11,
wherein the second connecting means (17, 17.1, 17.2) are formed as screw connection means.

13. The container according to claim 11 or 12, wherein means (18) for guaranteeing a first opening are provided.

14. The container according to claim 13,
wherein the means (18) for guaranteeing a first opening comprise a ring (19) that is arranged substantially unrotatably around the head (3) of the container body and that is connected via bars (20) to the rim (21) of the cap (4) such that the connection between the rim (21) and the ring (19) breaks when the cap (4) is rotated.
